# EUROPÄISCHE PATENTSCHRIFT

(11) **EP 1 166 726 B1**
(45) Veröffentlichungstag und Bekanntmachung des Hinweises auf die Patenterteilung: **19.01.2005**
(21) Anmeldenummer: 01115020.8
(22) Anmeldetag: 20.06.2001
(51) Int. Cl.: A61F 2/68, A61F 2/64

(54) **Brems-Kniegelenk**
Brake knee joint
Articulation de genou à freinage

(30) Priorität: 20.06.2000 DE 20010892 U
(43) Veröffentlichungstag der Anmeldung: 02.01.2002
(73) Patentinhaber: Medi Bayreuth Weihermüller & Voigtmann GmbH & Co. KG, 95448 Bayreuth (DE)
(72) Erfinder: Cheng, Chia-Pao, Shu Lin, Taipai Hsien (TW)
(74) Vertreter: Schuhmann, Albrecht

(56) Entgegenhaltungen:
- EP-A- 0 095 872
- EP-A- 0 658 697
- EP-A- 0 941 720
- WO-A-97/10781
- DE-A- 1 575 556
- DE-A- 3 309 233

## Beschreibung

Es ist bekannt, daß Behinderte seit kurzem künstliche Gliedmaßen benutzen, um sich leichter zu bewegen, wodurch sie laufen können, als ob sie Füße hätten. Deshalb wurden verschiedene Arten von Kniegelenkstrukturen zur Simulation der Bewegung eines Kniegelenks entwickelt, um diesen oben genannten Bedarf zu erfüllen. Künstliche Gliedmaßen sind jedoch mechanische Strukturen, und Menschen mit Beinbehinderungen müssen den Oberschenkel bewegen können, damit die künstlichen Gliedmaßen arbeiten. Gewöhnlich verwendete künstliche Gliedmaßen umfassen eine Druckvorrichtung, die eine Achse verwendet, so daß sie sich im Verhältnis zu einem Schaft drehen. Da die künstlichen Gliedmaßen jedoch nicht tatsächlich Teil des menschlichen Körpers sind, ist deren Kontrolle und gleichmäßige Bewegung schwierig. Künstliche Gliedmaßen drehen sich zu schnell, wenn sie beim Laufen gedreht werden. Wenn künstliche Gliedmaßen zu stark gebeugt werden können sie die Druckbuchse nicht durchlaufen, dadurchbewegten sich die künstlichen Gliedmaßen schnell in einen rechten Winkel.

Ein Kniegelenk mit unterschiedlichen Benge- und Strakgeschwindigkeiten ist aus WO 97/10781 bekannt.

Es ist eine Aufgabe der vorliegenden Erfindung, die Struktur eines Kniegelenks zu verbessern, wodurch die oben genannten Nachteile beseitigt und gemildert werden können.

Diese Aufgabe wird durch einen Kniegelenk gemäß Anspruch 1 gelöst.

Die beigefügten Zeichnungen zeigen:
- Fig. 1: eine auseinandergezogene Darstellung des erfindunggemäßen Kniegelenks;
- Fig. 2: einen Längsschnitt des erfindungsgemäßen Kniegelenks;
- Fig. 3 und 4: das Arbeitsprinzip des erfindungsgemäßen Kniegelenks;
- Fig. 5: das Herunterdrücken des Knopfes, um die Drehbewegung zu stoppen; und
- Fig. 6: eine auseinandergezogene Darstellung der Dreheinrichtung.

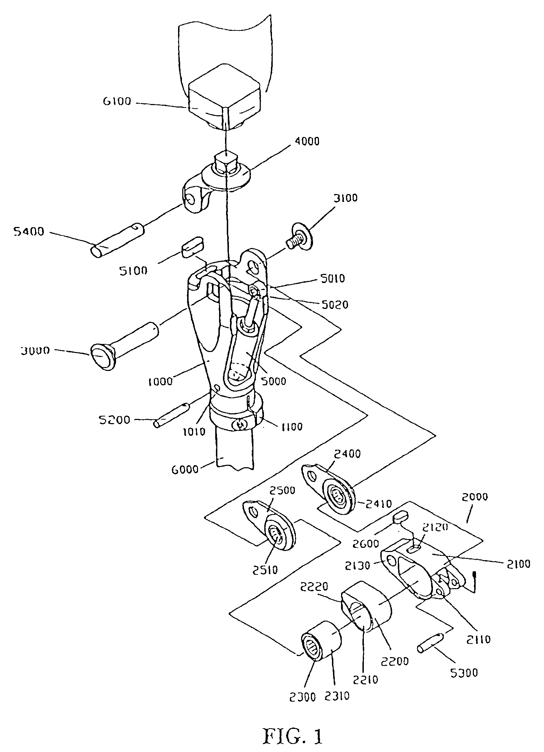

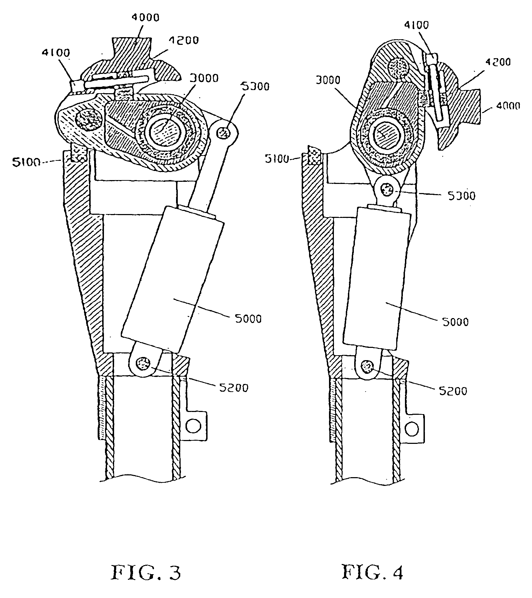

Wie in den Zeichnungen und insbesondere in Fig. 1 gezeigt, verwendet das erfindungsgemäße Kniegelenk grundsätzlich einen Schaft 3000, der ein rotierendes Teilstück 2000 in einem Grundkörperabschnitt 1000 hält, so daß das rotierende Teitstuck 2000 im Verhältnis zum Schaft 3000 gedreht werden kann. Am unteren Ende des Grundkörperabschnittes 1000 ist eine Buchse 1100 vorgesehen, die einen Stab 6000 hält, der schwenkbar mit dem Knöchel und dem Fuß verbunden wird. Eine Dämpfungseinrichtung 5000 ist durch einen Stift 5200, der durch ein Loch 1010 am unteren Ende des Gruhdicörperabschnitts 1000 verläuft, schwenkbar mit dem Grundkörperabschnitt 1000 verbunden, so daß die Dämpfungseinrichtung 5000 im Verhältnis zum Stift 5200 gedreht werden kann. Das andere Ende der Dämpfungseinrichtung 5000 ist durch einen Stift 5300, der durch die Löcher 2110 der Dreheinrichtung 2100 und ein Loch 5010 der Dämpfungseinrichtung 5000 geht, drehbar mit dem hinteren Ende der Dreheinrichtung 2100 verbunden, so daß beim Drehen der Dreheinrichtung 2100 im Uhrzeigersinn (siehe Fig. 3) die Dämpfungseinrichtung 5000 die Bewegung der Dreheinrichtung 2100 verlangsamt wodurch verhindert wird, daß der Anwender aus dem Gleichgewicht kommt, und er somit vor einer Gefahr geschützt wird. Auf der Oberseite des Grundkörperabschnitts 1000 ist außerdem ein Elastomer 5100 angebracht, um einen Stoß und einen Schlag zu absorbieren, die erzeugt werden, wenn sich das rotierende Teilstück 2000 zuruckdreht.

Ein Schwenkteil 6100 ist im Oberschenkel des Benutzers installiert, und eine Abdeckung 4000 ist mit dem Schwenkteil 6100 verbunden. Die Abdeckung 4000 ist durch einen Stift 5400, der durch das Loch 2130 der Dreheinrichtung 2100 geht, mit oder Dreheinrichtung 2100 verbunden.

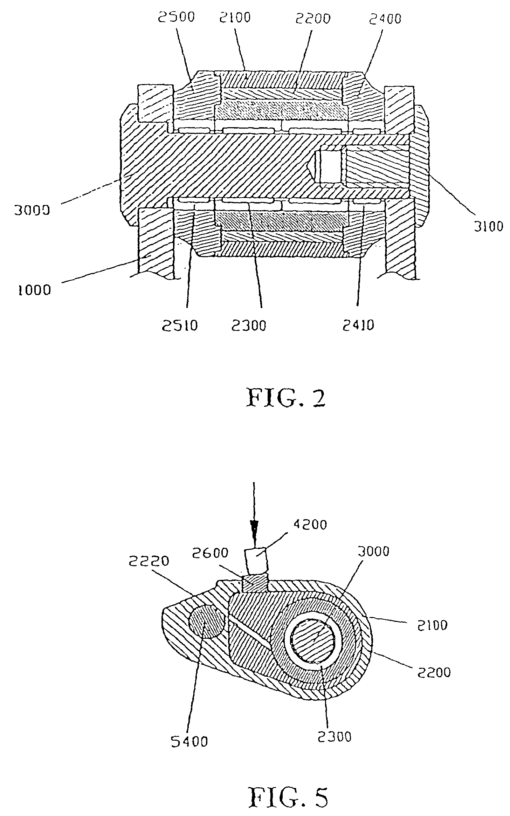

Siehe Fig. 2, eine Buchse 2200 und ein Einweglager 2300 sitzen in der Dreheinrichtung 2100 des rotierenden Teilstücks 2000, und zwei Ablenk- bzw. Prallteile (nachfolgend als Prallteile bezeichnet) 2400 und 2500 sind auf den beiden Enden der Dreheinrichtung 2100 befestigt. Ein Schaft 3000 erstreckt sich durch den Grundkörperabschnitt 1000 und die Dreheinrichtung 2100 und greift in eine Schraube 3100 ein, so daß die Dreheinrichtung 2100 im Verhältnis zum Schaft 3000 gedreht werden kann. Die Prallteile 2400 und 2500 sind mit Lagern 2410 bzw. 2510 versehen, damit die Drehbewegung des rotierenden Teilstücks 2000 gleichmäßig wird. Außerdem ist die Oberseite der Dämpfungseinrichtung 5000 mit einem Lager 5020 versehen, damit die Drehbewegung der Dämpfungseinrichtung 5000 gleichmäßig wird.

Siehe Fig. 3 und 4, wenn die Abdeckung 4000 im Verhältnis zum Schaft 3000 bis zu einer Position gedreht wird, in der sich die Dämpfungseinrichtung 5000 nicht mehr zusammenziehen kann, kann die Dämpfungseinrichtung 5000 die Rotationsgeschwindigkeit noch immer verringern. Innerhalb der Abdeckung 4000 befindet sich eine Schraube 4100 die in einen Druckstab 4200 eingreift, so daß das Drehen der Schraube 4100 die Position des Druckstabs 4200 regeln kann. Durch Einstellen der Position des Druckstabs 4200 kann die angewendete Kraft geregelt werden.

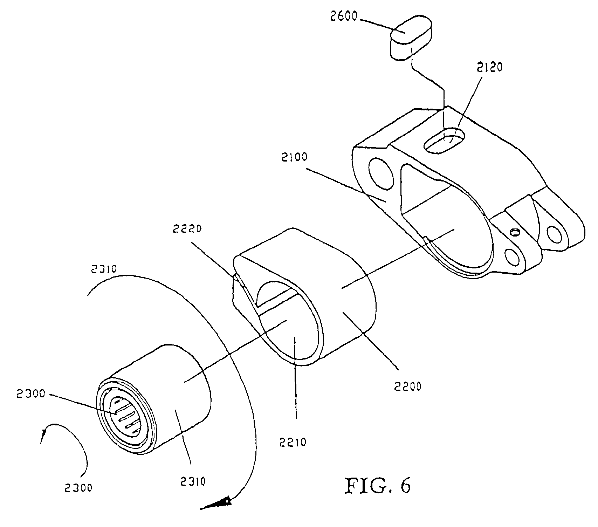

Wie in Fig. 5 und 6 gezeigt, dreht sich das Einweglager 2300 nur in einer Richtung, so daß sich das Eihweglager 2300 nur dann nur in einer Richtung, so daß sich das Einweglager 2300 nur dann dreht, wenn das rotierende Teilstück 2000 entgegengesetzt zum Uhrzeigersinn gedreht wird. Wenn das rotierende Teilstück 2000 im Uhrzeigersinn gedreht wird, kann sich das Einweglager 2300 nicht drehen, so daß die Außenoberfläche 2310 des Einweglagers 2300 im Gleichklang mit dem rotierenden Teilstück 2000 gedreht wird. Da sich die Außenoberfläche 2310 des Einweglagers 2300 innerhalb der Buchse 2200 dreht, nimmt die Reibung zwischen diesen zu, wodurch sich das rotierende Teilstück 2000 entgegengesetzt zum Uhrzeigersinn schnell, jedoch im Uhrzeigersinn langsam dreht. Außerdem ist die Dreheinrichtung 2100 an der Oberseite mit einer Öffnung 2120 versehen, in der ein Knopf 2600 sitzt. Wenn der Knopf 2600 heruntergedrückt wird, nimmt der Abstand 2220 der Buchse 2200 ab, wodurch die Reibung zwischen der Außenoberfläche 2310 des Einweglagers 2300 und der Innenoberfläche 2210 der Buchse 2200 zunimmt, wodurch die Drehbewegung gebremst wird. Somit muß nur wenig Kraft angewendet werden, um die Bewegung zu unterbrechen. Außer in der Position, in der das Drücken des Knopfes 2600 unmöglich ist, kann der Benutzer die Drehbewegung unterbrechen, selbst wenn der Winkel 30 Grad übersteigt.

## Patentansprüche

1. Kniegelenk, **gekennzeichnet durch** einen Schaft (3000), der ein rotierendes Teilstück (2000) in einem Grundkörperabschnitt (1000) anordnet, wodurch sich der Grundkörperabschnitt im Verhältnis zum Schaft drehen kann, wobei das rotierende Teilstück mit einem Einwegläger (2300) versehen ist, **durch** das sich das rotierende Teilstück schnell entgegengesetzt zum Uhrzeigersinn in seine ursprüngliche Position zurückbewegen kann, und das eine Dämpfungseinrichtung (5000) und eine stärkere Reibung anwendet, damit sich das rotierende Teilstück im Uhrzeigersinn langsam dreht, wodurch ein Behinderter künstliche Gliedmaßen bequem und leicht steuern und benutzen kann.

2. Kniegelenk nach Anspruch 1, **dadurch gekennzeichnet, daß** der Grundkörperabschnitt (1000) an der Oberseite mit einem Elastomer (5100) versehen ist, das einen Stoß und einen Schlag absorbiert, die erzeugt werden, wenn sich das rotierende Teilstück (2000) zurückdreht.

## Claims

1. A knee joint, **characterised by** a shaft (3000) which locates a rotating segment (2000) in a base body portion (1000), as a result of which the base body portion is able to turn in relation to the shaft, the rotating segment being provided with a one-way bearing (2300), by means of which the rotating segment is able to move back quickly anticlockwise into its original position, and which employs a damping device (5000) and a stronger friction so that the rotating segment turns slowly clockwise, enabling a disabled person to control and use artificial limbs comfortably and easily.

2. A knee joint according to Claim 1, **characterised in that** the base body portion (1000) is provided on the upper side with an elastomer (5 100) which absorbs a shock and an impact produced when the rotating segment (2000) turns back.

## Revendications

1. Articulation de genou, **caractérisé par** une tige (3000) qui place une pièce rotative (2000) dans une portion de corps de base (1000) pour permettre la rotation de la portion de corps de base par rapport à la tige, la pièce rotative étant dotée d'un palier unidirectionnel (2300) qui permet de ramener la pièce rotative rapidement dans le sens antihoraire à sa position de départ et qui recourt à un dispositif d'amortissement (5000) et à un frottement plus important pour que la pièce rotative tourne lentement dans le sens horaire pour permettre à une personne handicapée de commander et d'utiliser commodément et aisément des membres artificiels.

2. Articulation de genou selon la revendication 1, **caractérisé en ce que** la portion de corps de base (1000) est dotée du côté supérieur d'un élastomère (5100) qui absorbe un choc et une percussion qui sont générés lorsque la pièce rotative (2000) retourne en arrière.
